Europäisches Patentamt

European Patent Office

Office européen des brevets

(11) Veröffentlichungsnummer: **0 436 898 A1**

(12) **EUROPÄISCHE PATENTANMELDUNG**

(21) Anmeldenummer: 90124895.5

(22) Anmeldetag: 20.12.90

(51) Int. Cl.5: **A61B 5/02, A61M 25/00**

(30) Priorität: 22.12.89 DE 3942649

(43) Veröffentlichungstag der Anmeldung:
17.07.91 Patentblatt 91/29

(84) Benannte Vertragsstaaten:
**AT BE CH DE DK ES FR GB GR IT LI LU NL SE**

(71) Anmelder: **Kratzer, Michael, Dr.**
**Leopoldstrasse 56**
**W-8000 München 40(DE)**

(72) Erfinder: **Kratzer, Michael, Dr.**
**Leopoldstrasse 56**
**W-8000 München 40(DE)**

(74) Vertreter: **von Puttkamer, Nikolaus, Dipl.-Ing.**
**Pienzenauerstrasse 2**
**W-8000 München 80(DE)**

(54) **Einrichtung zur Untersuchung der Funktionen des Endothels bzw. Intimas von Blutgefässen.**

(57) Die Erfindung betrifft eine Einrichtung zur Untersuchung der Funktionen des Endothels bzw. des Intimas von Blutgefäßen. Ein Rohr (1) weist voneinander beabstandet in seiner Wandung wenigstens einen Ausgang (6) und einen Eingang (7) auf. Der Ausgang (6) steht mit einem Zugang (8) und der Eingang (7) steht mit einem weiteren Zugang (9) in Verbindung. Der Zugang (8) und der weitere Zugang (9) befinden sich an der aus dem Blutgefäß (4) herausragenden Seite des Rohres (1).

Fig. 1

EP 0 436 898 A1

# EINRICHTUNG ZUR UNTERSUCHUNG DER FUNKTIONEN DES ENDOTHELS BZW. INTIMAS VON BLUTGE-FÄSSEN

Die Erfindung betrifft eine Einrichtung zur Untersuchung der Funktionen des Endothels bzw. Intimas von Blutgefäßen nach dem Oberbegriff des Patentanspruches 1.

Bislang gibt es keine brauchbaren Methoden, um Funktionen des Endothels bzw. Intimas von Blutgefäßen in Vivo an Mensch oder Tier zu untersuchen.

Die Aufgabe der Erfindung besteht daher darin, eine Einrichtung zur Untersuchung der Funktionen des Endothels bzw. Intimas von Blutgefäßen zu schaffen, mit deren Hilfe Untersuchungen an gesunden, physiologischen Endothel- bzw. Intima-Zellen möglichen sind.

Diese Aufgabe wird durch eine Einrichtung der eingangs genannten Art gelöst, die durch die in dem kennzeichnenden Teil des Patentanspruches 1 angegebenen Merkmale gekennzeichnet ist.

Der wesentliche Vorteil besteht darin, daß mit der erfindungsgemäßen Einrichtung erstmals direkt Untersuchungen in Vivo an gesunden Endothel- bzw. IntimaZellen von Mensch und Tier möglich sind.

Vorteilhafterweise können durch die mit der erfindungsgemäßen Einrichtung ausgeführten Untersuchungen Erkenntnisse von wichtigen Funktionen des Endothels bzw. Intimas erhalten werden. Dadurch können beispielsweise Erkenntnisse über die Interaktion des Endothels mit dem Gerinnungssystem, d.h. also auch zum Beispiel der Thromboseneigung, der Blutungsneigung oder von arteriosklerotischen Frühveränderungen, gesammelt werden.

Vorzugsweise können mit der Hilfe der erfindungsgemäßen Einrichtung Tierversuche beliebig oft und für das Tier völlig schad- und schmerzlos ausgeführt werden.

Mit der Hilfe der Erfindung können wertvolle Erkenntnisse gewonnen werden, die für die Entwicklung von Medikamenten, die beispielsweise zur Behandlung der Arteriosklerose dienen, von wesentlicher Bedeutung sind.

Vorteilhafte Ausgestaltungen der Erfindung gehen aus den Unteransprüchen hervor.

Im folgenden werden die Erfindung und deren Ausgestaltungen im Zusammenhang mit den Figuren näher erläutert. Es zeigt:

Fig. 1     in schematischer Darstellung eine erfindungsgemäße Einrichtung zur Erläuterung der prinzipiellen Wirkungsweise der Erfindung; und

Fig. 2     bis 6 Ausführungsformen der erfindungsgemäßen Einrichtung.

Zu der Erfindung führten die folgenden Überlegungen. Um erstmals Untersuchungen in Vivo an Endothel- bzw. Intima-Zellen der Blutgefäße von Mensch und Tier zu ermöglichen ist es nötig, einen Zugang zu den die Blutgefäße auskleidenden Endothel- bzw. Intima-Zellen zu schaffen. Es wurde daher eine Einrichtung geschaffen, die katheterartig in ein Blutgefäß, beispielsweise eine Vene oder Arterie einschiebbar ist. An einer vorgegebenen Stelle wird dann ein Teilbereich des Blutgefäßes nach beiden Seiten dicht verschlossen. Durch Einführung einer Lösung in den genannten Teilbereich und durch Perfusion derselben im Teilbereich wird die eingebrachte Lösung in Kontakt mit den das Blutgefäß im Teilbereich auskleidenden Endothel- bzw. Intima-Zellen gebracht. Die Lösung wird schließlich aus dem Teilbereich abgesaugt und untersucht. Durch Vergleichsmessungen können Veränderungen der eingebrachten Lösung erfaßt werden. Aus den Veränderungen kann auf die Funktion der Endothel- bzw. Intima-Zellen geschlossen werden.

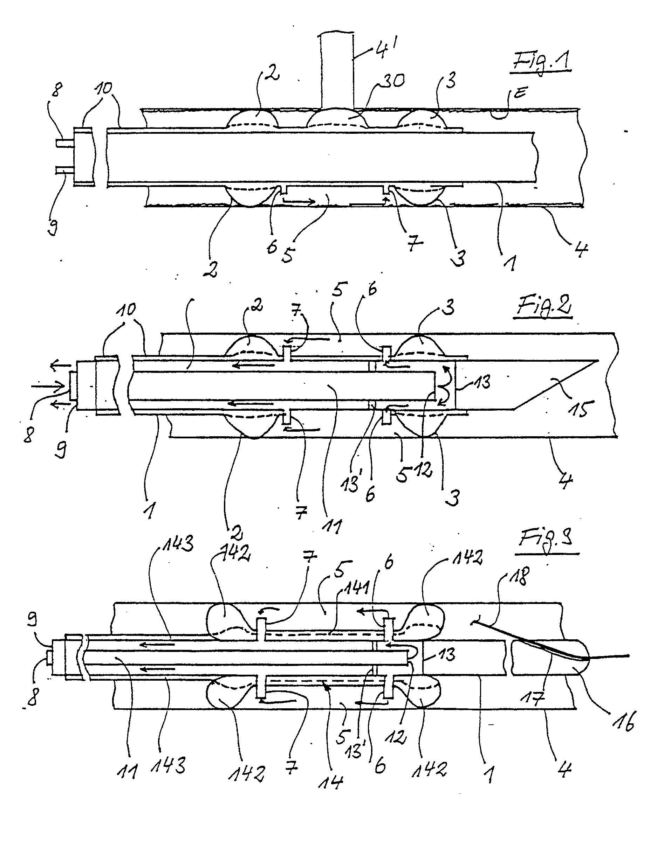

Gemäß Fig. 1 besteht die eine Ausführungsform der vorliegenden Erfindung im wesentlichen aus einem Rohr 1, an dem zwei voneinander beabstandete Vorrichtungen 2, 3 vorgesehen sind, die nach dem Einbringen des Rohres 1 in ein Blutgefäß 4 derart betätigt werden können, daß sie jeweils eine Abdichtung zwischen dem Umfang des Rohres 1 und der Innenwand des Blutgefäßes 4 bewirken. Auf diese Weise wird zwischen den Vorrichtungen 2 und 3 ein Bereich 5 geschaffen, der von dem Rest des Blutgefäßes 4 abgetrennt ist. An der Innenwandung des Blutgefäßes 4 befindet sich das Endothel E bzw. das Intima. Die vorliegende Einrichtung weist ferner zwischen den Vorrichtungen 2 und 3 wenigstens einen Ausgang 6 und wenigstens einen Eingang 7 auf, wobei ein Lösungsmittel durch den Ausgang 6 in den Bereich 5 einbringbar und über den Eingang 7 aus dem Bereich 5 wieder abziehbar ist. Vorzugsweise ist der Ausgang 6 in der Nähe der einen Vorrichtung 2 angeordnet, während sich der Eingang 7 in der Nähe der anderen Vorrichtung 3 befindet . Der Ausgang 6 und der Eingang 7 können beispielsweise die Form von die Wandung des Rohres 1 durchsetzenden stutzenförmigen Teilen oder von einfachen Öffnungen in der Wandung des Rohres 1 aufweisen. Das durch den Ausgang 6 eingebrachte Lösungsmittel durchströmt den Bereich 5 von dem einen zum anderen Ende. Dadurch, daß mehrere Ausgänge 6 und mehrere Eingänge 7 über den Umfang des Rohres 1 zweckmäßigerweise gleichmäßig verteilt werden, kann eine gleichmäßige Superfusion des Endothels bzw. Intimas im Bereich 5 mit dem Lösungsmittel bewirkt werden.

Das freiliegende Ende der Einrichtung weist zum Einbringen des Lösungsmittels einen Zugang 8, der zum Ausgang 6 führt, und einen Zugang 9 zur Entnahme des Lösungsmittels auf, der mit dem Eingang 7 in Verbindung steht.

Die genannten Vorrichtungen 2 und 3 werden vorzugsweise durch ringförmige, ballonartige Elemente gebildet, wie sie beispielsweise in anderem Zusammenhang, nämlich bei dem Herzkatheter nach Grünzig, bekannt sind. Genauer gesagt umgibt ein Kunststoffrohr 10 das Rohr 1 konzentrisch. Das Kunststoffrohr 10 weist als Vorrichtungen Zonen 2, 3 auf, die im Vergleich zu dem restlichen Kunststoffrohr 10 flexibler sind. Der Raum zwischen dem Rohr 1 und dem Kunststoffrohr 10 ist mit einem vorzugsweise flüssigen Medium ausgefüllt. Im Normalzustand entsprechen die Zonen 2, 3 den gestrichelten Linien. Wenn das genannte Medium unter Druck gesetzt wird, beulen sich die flexibleren Zonen 2 und 3 derart aus, daß sie eine Abdichtung zwischen der Einrichtung und dem Blutgefäß 4 jeweils bewirken. Wenn der Ausgang 6 und der Eingang 7 durch stutzenartige Teile gebildet werden, sind diese durch abgedichtete Ausnehmungen des Kunststoffrohres 10 hindurchgeführt. In dem Fall, in dem der Ausgang 6 und der Eingang 7 durch Öffnungen in der Wandung des Rohres 1 gebildet sind, befinden sich diese Öffnungen hinter abgedichteten Ausnehmungen des Kunststoffrohres 10.

Aus der Fig. 2 ist eine vorliegende Einrichtung ersichtlich, bei der die Verbindung zwischen dem Zugang 8 und dem Ausgang 6 durch ein Innenrohr 11 gebildet ist, dessen offenes Ende 12 vor dem verschlossenen Ende 13 des Rohres 1 angeordnet ist, so daß das einströmende Lösungsmittel aus dem offene Ende 12 zum Ausgang, 6 strömt, der wieder die Form einer Öffnung in der Wandung des Rohres 1 oder eines die Wandung des Rohres 1 stutzenartig dicht durchstzenden Teiles aufweist. Der Eingang 7 weist ebenfalls die Form einer Öffnung in der Wandung des Rohres 1 oder eines die Wandung des Rohres 1 stutzenartig durchsetzenden Teiles auf. Durch den Eingang 7 strömt das Lösungsmittel aus dem Bereich 5 in den Raum zwischen dem Innenrohr 11 und dem Rohr 1. Um eine Vermengung des durch den Ausgang 6 strömenden -Lösungsmittels mit dem durch den Eingang 7 strömenden Lösungsmittel zu vermeiden, ist eine Trennwand 13' vorgesehen, die den Zwischenraum zwischen dem Rohr 1 und dem Innenrohr 11 dicht verschließt. Die Trennwand 13' b efindet sich unterhalb des Bereiches 5 zwischen dem Eingang 6 und dem Ausgang 7, vorzugsweise in der Nähe des Einganges 6.

Aus der Fig. 3 geht eine Weiterbildung hervor, bei der die Vorrichtungen 2 und 3 durch ein aufblasbares, manschettenartiges Teil 14 gebildet sind, das das Rohr 1 ringförmig umgibt und einen relativ unflexiblen mittleren Bereich 141 und relativ flexible Endbereiche 142 aufweist. Das Teil 14 steht dicht mit einer Leitung 143 in Verbindung, die durch das Rohr 1 oder außen am Rohr 1 konzentrisch oder als an der Außenfläche des Rohres 1 befestigtes Leitungsteil verlaufen kann. In dem Fall, in dem die Leitung 143 durch das Rohr 1 verläuft, ist für eine dichte Durchtrittsstelle der Leitung 143 durch die Wandung des Rohres 1 gesorgt. Die unterbrochenen Linien zeigen das Teil 14 in der nicht expandierten Form. Das im Zusammenhang mit der Fig. 2 bereits genannte, unter Druck zu setzende Medium, befindet sich auch bei der Ausführungsform der Fig. 3 im Inneren des Teiles 14 und der Leitung 143. Der Eingang 6 und der Ausgang 7, die wieder als einfache Öffnungen in der Wandung des Rohres 1 oder als stutzenartige Teile au sgebildet sein können, sind unterhalb von abgedichteten Ausnehmungen des mittleren Teiles 141 angeordnet oder durch diese Ausnehmungen hindurchgeführt.

Gemäß Fig. 2 kann das vordere Ende des Rohres 1 mit einem sich schräg zu einer Spitze verjüngenden Endbereich 15 versehen sein, der ein besonders gutes Einführen in ein Blutgefäß 4 ermöglicht.

Gemäß Fig. 3 kann das Ende des Rohres 1 mit einem Kopfteil 16 versehen sein, das an seinem freien Ende abgerundet ist, so daß es besonders gut in einem Blutgefäß 4 vorwärtsgeschoben werden kann. Das Kopfteil 16 kann einen Durchgang 17 aufweisen, der sich vom freien Ende aus zu einer Seite des Kopfteiles 16 erstreckt. Vorzugsweise ist die eine Austrittsöffnung des Durchganges 17 mittig im feien Ende angeordnet. Durch den Durchgang 17 kann in an sich bekannter Weise, wie dargestellt, ein Führungsdraht 18 eingeschoben werden, der als Führung für die Einrichtung in einem Blutgefäß 4 dient.

Bei der Ausführungsform der Fig. 4 weist das ringförmige, manschettenartige Teil im aufgeweiteten Zustand einen Innendurchmesser auf, der größer ist als der Außendurchmesser des Rohres 1. Das Rohr 1 ist an der -Innenwandung des Teiles 14 befestigt. Im aufgeweiteten Zustand wird daher ein Raum 20 zwischen dem Rohr 1 und dem Teil 14 geschaffen, durch den Blut beispielsweise in der Richtung des Pfeiles 21 hindurchsströmen kann, wenn sich die Einrichtung in einem Blutgefäß 4, beispielsweise eine Arterie, befindet. Dies bedeutet, daß der Bereich 5 von der Innenwandung der Arterie 4 abgetrennt werden kann, ohne daß der Blutfluß durch die Arterie unterbrochen wird.

Allgemein kann die zuvor angesprochene Blutströmung bei den Ausführungsformen der Fig. 1 bis 3 auch dadurch erreicht werden, daß eine Überbrückungsleitung zwischen Orten vorgesehen wird, die an gegenüberliegenden Seiten außerhalb der Zonen 2, 3 bzw. des Teiles 14 liegen. In Fig. 1 ist der Verlauf einer derartigen Überbrückungsleitung beispielhaft durch die strichpunktierte Linie 23 angedeutet.

Bei allen beschriebenen Ausführungsformen ist es denkbar, im Bereich 5 eine weitere flexible Zone 30 vorzusehen (Fig. 1), die sich nicht ringartig über den gesamten Umfang, sondern nur über einen Teilbereich des Umfanges erstreckt. Durch diese Zone 30 können Abzweigungen an Blutgefäßen 4' im Bereich 5 abgedichtet werden. Derartige Abzweigungen könnten die vorzunehmenden Untersuchungen stören. Es ist auch denkbar, die Länge der Ausnehmung 30 in der Richtung der Längserstreckung des Rohres 1 so zu bemessen, daß die Ausnehmung 30 in dem Teilbereich des Umfanges offen in die Zonen 2 und 3 übergeht.

Eine besonders einfache Einrichtung der Erfindung geht aus der Fig. 5 hervor. Demgemäß besteht die Einrichtung lediglich aus einem Rohr 1, das voneinander beabstandet wenigstens einen Eingang 6 und wenigstens einen Ausgang 7 aufweist, die zweckmäßigerweise durch Öffnungen in der Wandung des Rohres 1 gebildet sind. Die Abdichtung wird in diesem Falle von außen durch Abklemmen des Blutgefäßes 4 außerhalb des Ein- und Ausganges 6, 7 mit einer Vorrichtung vorgenommen, die beispielweise zwei an einem Träger voneinander beabstandet befestigte klingenartige Druckelemente aufweist. Die Verbindungen zwischen den Zugängen 8 und 9 und dem Eingang 6 bzw. dem Ausgang 7 kann auch hier beliebig, beispielsweise in der im Zusammenhang mit den Fig. 2 und 3 beschriebenen Weise erfolgen.

Vorzugsweise bestehen bei den Ausführungsformen der Fig. 1 bis 5 das Rohr 1 aus Metall, das Innenrohr 11 aus Metall oder Kunststoff, das Rohr 10 (Fig. 1 und 2) aus Kunststoff mit Bereichen unterschiedlicher Flexibilitäten, die Leitung 143 (Fig. 3 und 4) aus Kunststoff und das Teil 14 (Fig. 3 und 4) aus Kunststoff mit Bereichen unterschiedlicher Flexibilitäten.

Bei dem als Ende 13 bezeichneten Teil kann es sich um eine den Hohlraum des Rohres 1 abdichtende Querwand handeln, die im Endbereich oder an einer anderen Stelle des Rohres 1 angeordnet ist.

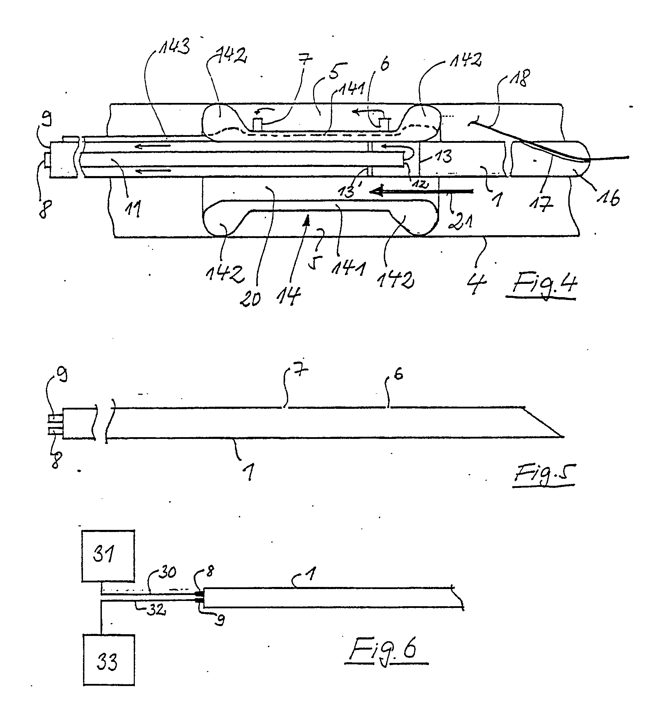

Aus der Figur 6 ist ersichtlich, daß der Zugang 8, durch den die genannte Lösung eingebracht wird, über eine Leitung 30 mit einem Reservoir 31 verbunden ist, in dem sich ein Vorrat der Lösung befindet. Der Zugang 9 ist über eine weitere Leitung 32 mit einer Analysiereinrichtung 33 verbunden. Beim Betrieb wird dem Reservoir 31 Lösung entnommen und über die Leitung 30 und den mit dem wenigstens einen Ausgang 6 verbundenen Zugang 8 dem Bereich 5 zugeführt und in diesem durch Perfusion in Kontakt mit dem das Blutgefäß im Bereich 5 auskleidenden Endothel- bzw. Intima-Zellen gebracht. Die Lösung wird über den Eingang 7 dem Zugang 9 zugeführt und gelangt über die Leitung 32 zur Analysiereinrichtung 33. Diese untersucht, inwiefern sich die ihr über den Zugang 9 zugeführte Lösung durch den Kontakt mit den Endothel- bzw. Intima-Zellen verändert hat. Hierzu vergleicht die Analysiereinrichtung 33 die ihr über die Leitung 32 zugeführte Lösung mit der den Reservoir 31 entnommenen Lösung. Die Erfindung umfaßt somit auch e ine Einrichtung, bestehend aus dem Reservoir 31, der katheterartigen Einrichtung und der Analysiereinrichtung 33 in Kombination, wobei die genannten Verbindungen zwischen den Zugängen 8, 9 und den Leitungen 30, 32 bestehen.

Zum Beispiel kann man in der einfließenden Lösung Vasopressinanalog (DDAVP) zugeben, das auf die Endothelzellen wirkt, so daß diese von Willebrand Faktor freisetzen, der dann durch die Analysiereinrichtung gemessen wird.

## Patentansprüche

1. Einrichtung zur Untersuchung der Funktionen des Endothels bzw. des Intimas von Blutgefäßen, dadurch gekennzeichnet, daß ein Rohr (1) voneinander beabstandet in seiner Wandung wenigstens einen Ausgang (6) und einen Eingang (7) aufweist, daß der Ausgang (6) mit einem Zugang (8) und der Eingang (7) mit einem weiteren Zugang (9) in Verbindung stehen, und daß sich der Zugang (8) und der weitere Zugang (9) an der aus dem Blutgefäß (4) herausragenden Seite des Rohres (1) befinden.

2. Einrichtung nach Anspruch 1, dadurch gekennzeichnet, daß seitlich neben dem Ausgang (6) und dem Eingang (7) außerhalb des durch den Ausgang (6) und dem Eingang (7) umschriebenen Bereiches jeweils eine Vorrichtung (2, 3) angeordnet ist, die wahlweise eine dichte Verbindung zwischen dem Außenumfang des Rohres (1) und der Innenwandung des Blutgefäßes (4) herstellen kann.

3. Einrichtung nach Anspruch 2, dadurch gekennzeichnet, daß ein Kunststoffrohr (10) das Rohr (1) konzentrisch umgibt, das zur Bildung der Vorrichtungen (2, 3) voneinander beabstandet ringförmige Zonen aufweist, in denen das Material des Kunststoffrohres (10) flexibler ist als das Material des Kunststoffrohres (10) in den restlichen Bereichen und/oder in denen der Umfang des Kunststoffrohres (10) größer ist als in den restlichen Bereichen, so daß die Zonen zur Herstellung der dichten Verbindungen ausbeulen, wenn ein im Kunststoffrohr (10) befindliches Medium unter Druck gesetzt wird.

4. Einrichtung nach Anspruch 3, dadurch gekennzeichnet, daß zwischen den Zonen (2, 3) eine weitere Zone (30) vorgesehen ist, die sich nur über einen Teilbereich des Umfanges des Kunststoffrohres (10) erstreckt und aus dem vergleichsweise flexibleren Material besteht und/oder in der der Umfang des Kunststoffrohres (10) größer ist als in den restlichen Bereichen.

5. Einrichtung nach Anspruch 2, dadurch gekennzeichnet, daß als Vorrichtung auf dem Rohr (1) ein ringförmiges, manschettenartiges Kunststoffteil (14) angeordnet ist, das einen mittleren Bereich (141) und vergleichsweise flexiblere und/oder gegenüber dem mittleren Bereich (141) einen größeren Durchmesser aufweisende Endbereiche (142) aufweist, daß das Teil (14) mit einer Leitung (143) derart dicht verbunden ist, daß ein in der Leitung (143) und dem Teil (14) befindliches Medium derart unter Druck setzbar ist, daß die Endbereiche (142) durch Ausbeulungen die dichten Verbindungen herstellen.

6. Einrichtung nach Anspruch 5, dadurach gekennzeichnet, daß die Leitung (143) das Rohr (1) konzentrisch umgibt, außen an dem Rohr (1) verläuft oder im Rohr (1) verläuft und dicht durch dieses hindurchgeführt ist.

7. Einrichtung nach Anspruch 5, dadurch gekennzeichnet, daß der Innendurchmesser des ringförmigen Teiles (14) dann, wenn das Medium unter Druck steht, größer ist als der Außendurchmesser des Rohres (1), so daß ein Durchgang für Blut zwischen dem Rohr (1) und dem Teil 814) gebildet wird.

8. Einrichtung nach Anspruch 7, dadurch gekennzeichnet, daß die Leitung (143) außen am Rohr (1) verläuft oder im Rohr (1) verläuft und dicht durch dieses hindurchgeführt ist.

9. Einrichtung nach einem der Ansprüche 1 bis 8, dadurch gekennzeichnet, daß im Rohr (1) ein Innenrohr (11) angeordnet ist, dessen im Rohr (1) befindliches Ende (12) verschlossen und von einer den Hohlraum des Rohres (1) abdichtenden Querwand (13) des Rohres (1) beabstandet ist, daß von der Querwand (13) des Rohres (1) aus gesehen hinter dem Ende (12) des Innenrohres (11) eine Trennwand (13') angeordnet ist, die den Zwischenraum zwischen dem Innenrohr (11) und dem Rohr (1) abdichtet, daß der Ausgang (6) in den Raum zwischen der Trennwand (13') und der Querwand (13) führt und daß der Eingang (7) von der Querwand (13) des Rohres (1) aus gehen in dem Bereich hinter der Trennwand (13') angeordnet ist.

10. Einrichtung nach einem der Ansprüche 1 bis 9, dadurch gekennzeichnet, daß das Rohr (1) einen in das Blutgefäß (4) einzuführenden Endbereich (15) aufweist, der sich schräg zu einer Spitze verjüngt.

11. Einrichtung nach einem der Ansprüche 1 bis 9, dadurch gekennzeichnet, daß der in das Blutgefäß (4) hineinragende Endbereich des Rohres (1) die Form eines an seinem freien Ende abgerundeten Kopfteiles (16) aufweist.

12. Einrichtung nach Anspruch 11, dadurach gekennzeichnet, daß das Kopfteil (16) einen Durchgang aufweist, der sich vom freien Ende aus zu einer Seite des Kopfteiles (16) erstreckt und daß durch den Durchgang (17) ein Führungsdraht (18) schiebbar ist.

13. Einrichtung nach einem der Ansprüche 1 bis 12, dadurch gekennzeichnet, daß über jeweils einen Umfangsbereich des Rohres (1) mehrere Ausgänge (6) bzw. Eingänge (7) vorgesehen sind.

14. Einrichtung nach einem der Ansprüche 1 bis 13, dadurch gekennzeichnet, daß der Ausgang (6) und der Eingang (7) die Form einer Öffnung in der Wandung des Rohres (1) aufweist und daß gegebenenfalls das Kunststoffrohr (10) im Bereich dieser Öffnungen abgedichtete Ausnehmungen aufweist.

15. Einrichtung nach einem der Ansprüche 1 bis 13, dadurch gekennzeichnet, daß der Ausgang (6) und der Eingang (7) jeweils die Form eines die Wandung des Rohres (1) durchsetzenden stutzenartigen Teiles aufweisen und daß gegebenenfalls das Kunststoffrohr (10) im Bereich dieser stutzenartigen Teile abgedichtete Ausnehmungen aufweist.

16. Einrichtung nach einem der Ansprüche 1 bis 15, dadurch gekennzeichnet, daß das Rohr (1) aus Metall besteht.

17. Einrichtung nach einem der Ansprüche 1 bis 16, dadurch gekennzeichnet, daß das Innenrohr (11) aus Kunststoff oder Metall besteht.

18. Einrichtung nach einem der Ansprüche 1 bis 17, dadurch gekennzeichnet, daß der Zugang (8) über eine Leitung (30) mit einem Reservoir (31) für eine Lösung verbunden ist, daß der weitere Zugang (9) über eine weitere Leitung (32) mit einer Analysiereinrichtung (33) verbunden ist, die die Änderungen ermittelt, die zwischen der dem Reservoir (31) entnommenen und über den Zugang (8) zugeführten Lösung und der der Analysiereinrichtung (33) über den weiteren Zugang (9) zugeführten Lösung bestehen.

Fig.1

Fig.2

Fig.3

Fig.4

Fig.5

Fig.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | DE - A1 - 2 705 393 (HOSPAL) * Ansprüche 1-4,24-26; Fig. 1-3 * | 1,10, 13,14, 16-18 | A 61 B 5/02 A 61 N 25/00 |
| A | -- | 9 | |
| X | FR - A1 - 2 297 640 (RHONE POULENC) * Seite 4, Zeilen 15-34; Fig. 4 * | 1-3,5, 6,8,9, 11,13, 14, 16-18 | |
| X | AU - B - 26 165/84 (VAS-CATH) * Anspruch 1; Fig. 3-6,8 * | 1,9, 10,13, 17 | |
| X | EP - A1 - 0 168 136 (SHILEY) * Zusammenfassung; Fig. 1 * | 1,9, 11,13, 14,17, 18 | |
| A | -- | 12 | |
| X | EP - A1 - 0 150 462 (AIGNER) * Zusammenfassung; Fig. 1,2 * | 1,9, 11,13, 14,17, 18 | RECHERCHIERTE SACHGEBIETE (Int Cl⁵) A 61 B A 61 M |
| A | -- | 12 | |
| X | EP - A1 - 0 101 890 (AIGNER) * Zusammenfassung; Fig. 2-3a * | 1,2,9, 11,13, 17,18 | |
| X | EP - A1 - 0 025 704 (SORENSON) * Fig. 2-5 * | 1 | |
| A | EP - A2 - 0 050 983 (EVANS) * Fig. 1,4,8 * | 2-8 | |

Der vorliegende Recherchenbericht wurde für alle Patentansprüche erstellt.

| Recherchenort WIEN | Abschlußdatum der Recherche 15-04-1991 | Prüfer NEGWER |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| | -- | | |
| A | US - A - 4 705 502 (PATEL) * Zusammenfassung; Fig. 1,2 * | 1-18 | |
| | -- | | |
| X | US - A - 4 610 662 (WEIKL) * Fig. 1-4 * | 1-18 | |
| | -- | | |
| X | WO - A1 - 88/03 389 (HÅLLGREN) * Seite 7, Zeile 18 - Seite 9, Zeile 26; Fig. 1 * | 1-18 | |
| | -- | | |
| A | WO - A1 - 86/05 378 (NICHOLSON) * Zusammenfassung; Fig. 1 * | 1,12 | |
| | ---- | | |

RECHERCHIERTE
SACHGEBIETE (Int Cl⁵)

Der vorliegende Recherchenbericht wurde für alle Patentansprüche erstellt.

| Recherchenort WIEN | Abschlußdatum der Recherche 15-04-1991 | Prüfer NEGWER |
|---|---|---|